# EUROPEAN PATENT SPECIFICATION

(11) **EP 0 784 460 B1**
(45) Date of publication and mention of the grant of the patent: **28.07.2004**
(21) Application number: 95939522.9
(22) Date of filing: 05.10.1995
(51) Int. Cl.: A61F 13/15, A61F 13/56

(54) **ABSORBENT SANITARY ARTICLE WITH EXPANDABLE FLAPS**
DAMENBINDE MIT DEHNBAREN LASCHEN
ARTICLE HYGIENIQUE ABSORBANT A VOLETS EXTENSIBLES

(30) Priority: 07.10.1994 IT TO940803
(43) Date of publication of application: 23.07.1997
(73) Proprietor: THE PROCTER & GAMBLE COMPANY, Cincinnati, Ohio 45202 (US)
(72) Inventor: PALUMBO, Gianfranco, D-41348 Bad Homburg (DE); SIERRI, Giancarlo, I-65015 Montesilvan (IT)
(74) Representative: Veronese, Pancrazio
(86) International application number: PCT/US1995/013272
(87) International publication number: WO 1996/010977

(56) References cited:
- EP-A- 0 426 235
- EP-A- 0 575 611
- WO-A-93/01782
- WO-A-93/06805
- WO-A-95/08311
- US-A- 4 589 876
- US-A- 4 911 701
- US-A- 4 940 462

## Description

This invention relates to absorbent sanitary articles.

Absorbent articles such as sanitary napkins, pantiliners, and incontinence pads are devices that are typically worn in the crotch region of an undergarment. These devices are designed to absorb and retain liquid and other discharges from the human body and to prevent body and clothing soiling. Sanitary napkins are a type of absorbent article worn by women in a pair of panties that is normally positioned between the wearer's legs, adjacent to the perineal area of the body. Sanitary napkins both with and without side flaps (or wings) are disclosed in the literature and are available in the marketplace. Some particularly preferred sanitary napkins that do not require flaps are described in US-A-4950264 and US-A-5009653.

Generally when sanitary napkins are provided with flaps, the flaps extend laterally from a central absorbent means and are intended to be folded around the edges of the wearer's panties in the crotch region. Thus, the flaps are disposed between the edges of the wearer's panties in the crotch region and the wearer's thighs. Commonly, the flaps are provided with an attachment means for either affixing the flaps to the underside of the wearer's panties or to the opposing flap. The flaps are particularly effective for preventing exudates from soiling the edges of the wearer's panties.

Sanitary napkins having flaps of various types are disclosed in US-A-4687478, US-A-4608047, US-A-4589876, US- A.4285343, US-A-3397697, US-A-2787271.

While sanitary napkins having flaps are commonly viewed as providing better protection against soiling as compared to sanitary napkins without flaps, some women find applying sanitary napkins having flaps to be inconvenient for various reasons. For instance, some women find it to be difficult to attach the flaps to the underside of the crotch of their panties. This can be due to factors such as the tendency for the adhesive fasteners on the flaps to stick to themselves or to other parts of the sanitary napkin. As a result, some women still prefer a sanitary napkin without flaps, and some women who generally prefer a sanitary napkin with flaps, occasionally (such as during periods of light flow) prefer a sanitary napkin without flaps. Therefore, there is a need for a sanitary napkin which provides an alternative to sanitary napkins having conventional side flaps while still providing the protection of side flaps.

In addition, both sanitary napkins with and without flaps are subject to the problem that the wearer's undergarments move with the wearer's movements during wear. Most sanitary napkins, however, provide no mechanism for adjusting to these movements. This puts stresses on the sanitary napkin and the flaps. These stresses may cause the sanitary napkin to shift from its desired position in the wearer's undergarment. The failure to provide the sanitary napkin with a mechanism to adjust to the difference between the movement of the wearer's undergarments and the wearer's body may also cause the sanitary napkin to be less comfortable than if it stretched and conformed with the wearer's movements and to the wearer's undergarments.

Several variations of sanitary napkins having conventional flaps that attempt to solve some, but not all of these problems are disclosed in the patent literature. For example, US-A-4911701 (Mavinkurve) discloses a sanitary napkin having elastic strands for providing a greater convex shape to the body-facing portion of the central absorbent and for enabling adhesive-free placement of the flaps of a winged napkin embodiment into a pair of panties. The sanitary napkins described in the Mavinkurve patent, however, still appears to require the user to manipulate the flaps (by first flipping the flaps upward and then placing the flaps in her panties and flipping the flaps back down) since the flaps appear to be pre-disposed to be in a downward folded condition. The Mavinkurve patent also requires that individual elastic strands be attached in a contracted condition to the central absorbent portion of the napkin and/or to its wings or flaps. The napkins described in the Mavinkurve patent can, therefore, be difficult and expensive to manufacture. The Mavinkurve patent also does not disclose any mechanism to account for differences in movement and extensibility of the central absorbent of the napkin and the wearer's panties.

US-A-4940462 (Salerno) discloses a sanitary napkin with longitudinally expandable flaps. The flaps are designed to fold over the exterior of the wearer's panty and then to expand to conform with the contour of the panties. The Salerno patent, however, appears to require conventional adhesive fasteners to retain the flaps in place on the underside of the wearer's panties. Further, in order for the Salerno flaps to extend an amount sufficient to wrap around the panty elastics, the flaps have to have a resiliently wide transverse dimension. The extra flap material can extend too far outward beyond the panty elastics to create a sloppy border that hangs out of the wearer's panties.

WO 93/06895 discloses a sanitary napkin with flaps having zones of differential extensibility for relieving the stresses that develop in the flaps when the flaps are folded down in use around the edges of the crotch portion of the wearer's undergarment.

It an object of the present invention to provide an absorbent article, such a sanitary napkin, which overcomes, or at least mitigates, some or all of the problems described above.

According to the present invention there is provided an absorbent sanitary article which comprises an absorbing region capable of absorbing body fluids and having a pair of side regions which are to form side flaps located one on each of two opposite edges of the absorbing region, each flap being non-planar and having a first edge, nearer the absorbing region, which edge defines a non-rectilinear axis of rotation for the flap to pivot with respect to the absorbing region, and a second edge, remote from the said absorbing region, where the perimetral length is greater than for an equivalent planar flap.

In manufacturing the article each of said regions is subjected to a stretching force both in a longitudinal direction and in a transverse direction, in order to achieve the non-planar form of said flaps. The material of the flaps is a non-elastic plastics material or combination of plastics materials, such that the material can be stretched in the course of manufacture of the article, but is not readily stretchable in use.

It is to be understood that the said first edge of each flap may be at, or substantially parallel to, a correspondingly non-rectilinear edge of the absorbing region, as is the case in the embodiment described below with reference to the drawings. Alternatively, however, the said first edge may be theoretical rather than actual in the article as manufactured. The side edges of the absorbing region may, for example, be straight, with the said first edges being within an area which lies outside the boundary of the absorbing material. In either event, however, the said first edges are those about which the flaps pivot in use with respect to the rest of the article which includes the said absorbing region.

The invention will now be further described with reference to the accompanying drawings in which:
Figure 1 is a plan view, seen from the topsheet side, of a blank from which an embodiment of the invention in the form of a sanitary napkin is to be made, showing the extent to which different regions of the article are to be stretched;
Figure 2 is a plan view of a sanitary napkin made from the blank of Figure 1;
Figure 3 is a side view of the sanitary napkin of Figure 2;
Figures 4, 5 and 10 show three alternative patterns in which a panty fastening adhesive can be applied to an article according to the present invention, again in the form of a sanitary napkin; and
Figures 6 to 9 are diagrammatic cross-sections through four alternative embodiments of the invention, as applied to sanitary napkins.

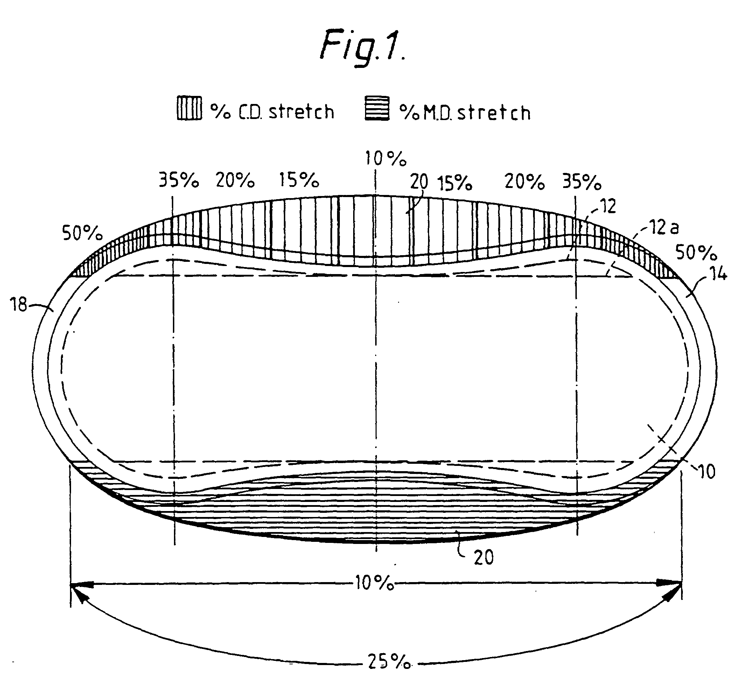

Referring first to Figure 1, this shows the blank from which a sanitary napkin according to the present invention can be formed. It will be seen that this comprises an absorbent core 10, which has straight, parallel longitudinal sides and rounded ends, and a secondary topsheet 12 which is of hourglass shape and which overlies the core 10. The ends of the secondary topsheet coincide with the ends of the core 10, but there are additional corner portions 12a below which no core 10 is present.

A primary topsheet 14 of generally elliptical shape is placed on the secondary topsheet, and a backsheet of the same shape is located below the core 10. Both the primary topsheet 14 and the secondary topsheet 12 are liquid- permeable, though the topsheet 14 may only be permeable in a central region thereof, and may be impermeable in a peripheral region thereof. The backsheet must be liquid- impermeable.

The backsheet and topsheet are sealed to one another, preferably by a heat seal, though alternatively by adhesive, along a band 18 which has the same shape as the periphery of the secondary topsheet 12 and is spaced outwardly therefrom. The secondary topsheet 12 and core 10 are thus encapsulated between the topsheet 14 and the backsheet 16.

A pair of side regions 20 which are to form side flaps, are constituted by portions of the topsheet/backsheet assembly which lie outwardly of the core and secondary topsheet. In each of these regions 20, the backsheet and topsheet are sealed to one another by adhesive extending over the mutually facing surfaces thereof. It would, however, be alternatively possible for the topsheet and backsheet to be secured to one another in the regions 20 by other means, for example by means of additional heat seals on the laterally outer edges thereof. In manufacturing the napkin, each of these regions is subjected to stretching both in a longitudinal direction (i.e. parallel to the longer axis of the napkin) and in a transverse direction (i.e. in a direction at right angles to the above longitudinal direction). In a conventional process for producing sanitary napkins, which can be modified to produce napkins according to the present invention, the webs of raw material to form the topsheets and backsheets travel through the manufacturing process in a direction parallel to the longitudinal axis of the articles, and this direction is therefore conventionally referred to as the machine direction (M.D.). The direction at right angles to this is therefore conventionally referred to as the cross direction (C.D.). In the ensuing description reference will be made to machine direction (M.D.) and cross direction (C.D.), but it must be understood that what is significant for present purposes are the directions with reference to the article, rather than with reference to the machine by which the article may be formed, and that M.D. and C.D. are therefore to be understood in the present context as referring primary to a direction parallel to the longitudinal axis of the napkin, and a direction which is transverse to this, respectively.

As already indicated, each of the regio 20 is subjected both to C.D. stretch and M.D. stretch. For ease of illustration, the extent of C.D. stretch is shown in only in relation to one of the regions, and the extent of M.D. stretch is shown only in relation to the other of the regions. It must be emphasised, however, that, in reality, both regions are subjected to both forms of stretching.

Referring first to the upper of the two regions 20 illustrated in Figure 1, this is subjected to an amount of stretching which varies from 10% at the longitudinal middle to 50% at the longitudinal ends. The degree of stretching is shown as being in bands of 10%, 15%, 20%, 35% and 50%. These bands are shown for illustrative purposes only, and in fact the amount of stretching may vary at least approximately continuously over the length of the flap. To a first approximation, it can be assumed that the values of 10%, 15%, 20% and 35% are achieved around the middle of the bands which are labelled with those values, and the value of 50% is achieved at the extreme ends. It should be understood that although the percentage stretch varies substantially as between the middle of the flap and its ends, the actual amount of stretch does not vary to a corresponding extent, since the amount of material available to be stretched is much greater at the centre than it is at the ends. The range of CD stretch may in fact be less than, or greater than, that illustrated in Figure 1. For example, it may range from less than 10% at the middle (even down to zero at that point) up to as much as about 70% at the ends.

The lower region 20 in Figure 1 has marked on it a uniform amount of M.D. stretch (10%). This is only an approximation, and can be taken as the average value of the stretch as considered from the region of the flap laterally inwards to the region of the flap laterally outwards. Laterally inwards, the amount of M.D. stretch may tail off almost, or completely, to zero. Laterally outwards, the amount of M.D. stretch reaches a value which exceeds 10%, and may indeed reach a value nearer 20%, or even up to about 40%.

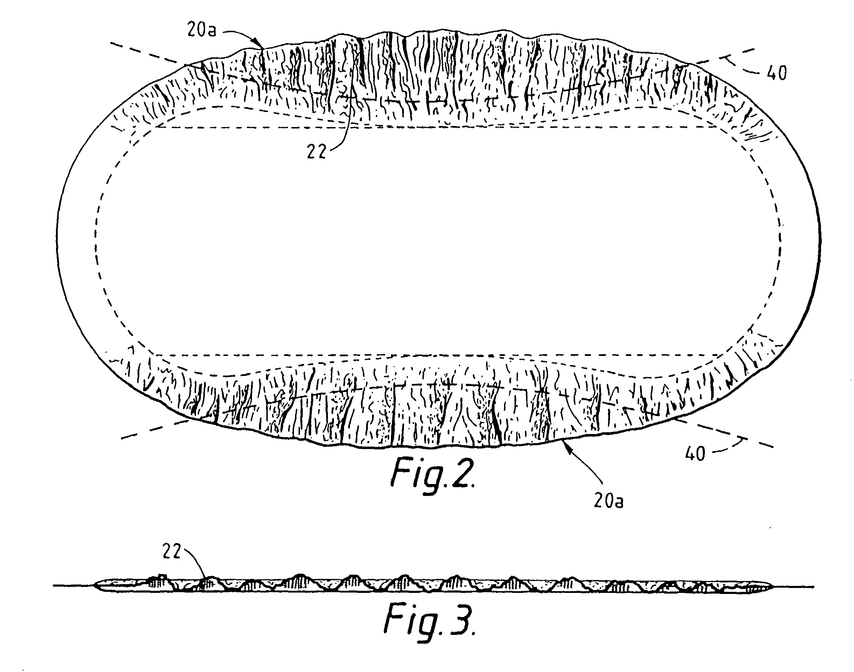

The result of the combined C.D. and M.D. stretch is to cause undulations or waves 22 to be formed in the regions 20, thus creating flaps 20a. These can be seen in Figures 2 and 3. The undulations 22 have their greatest amplitudes at their laterally outer ends, falling to zero, or almost to zero, at their inner ends. The periodicity of the undulations 22 is approximately constant from end to end. The effect of the undulations 22 is that the length of the laterally outer edge of each of the flaps 20a, as measured along a path which follows the rise and fall of the undulations, is increased compared to what it would have been had no undulations been formed. Preferably, the resulting increase in the perimetral length of each flap is from 10 to 70%, more preferably from 15 to 50%, and still more preferably from 20 to 30%. Most preferably, this increase is about 25%.

The materials of which the topsheet and backsheet are formed must, therefore, be such as are capable of being stretched. Furthermore, the materials are so chosen that, at least in their stretched condition, they are not elastic, or at least not elastic to any significant extent. In addition, the materials are chosen to be such that, although they can be stretched in the course of manufacture of the article, they are not readily stretchable thereafter. By this is meant that they are not such as will stretch to any significant extent during normal use, though this is not to say that a user could not effect some stretching of the materials if, contrary to what is intended, the user were to attempt to do so.

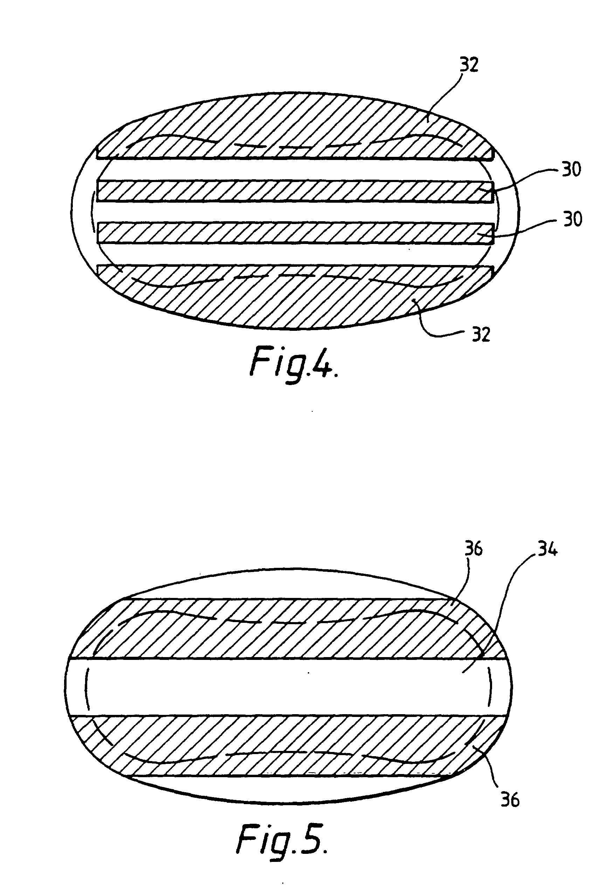

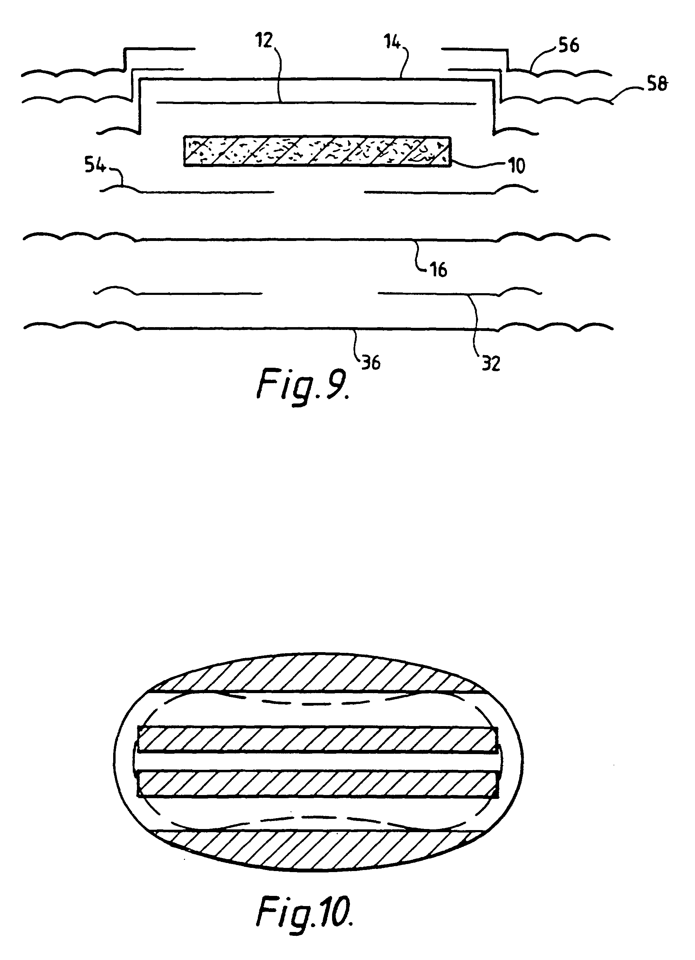

The regions 20 of the blank shown in Figure 1 may be stretched by any suitable method. However, a preferred method is as described in our copending Italian Patent Application filed on the same date as the present application and entitled [here insert title of DR63]. This describes a method of deforming a sheet-type region of an absorbent sanitary article, wherein the said region is positioned between a mould member having at least one aperture or recess therein, and preferably a plurality of such apertures or recesses, and a deforming member, the deforming member forcing the said region into the said aperture or recess. Preferably, the deforming member comprises a resilient membrane, and movement of the resilient member is effected by a fluid which exerts a pressure thereon. A portion of the article around said region is clamped during movement of the resilient member. The backsheet 16 has panty fastening adhesive applied thereto. The backsheet, with its adhesive, is normally covered by a protective release layer, which is removed by the user immediately before the article is worn. Figures 4 and 10 each show an adhesive pattern which comprises two parallel central stripes 30 and two laterally outward stripes 32, the latter extending to the outer edges of the article. In Figure 4 the stripes 32 cover, inter alia, the flaps 20a and in Figure 10 they cover only the flaps. Figure 5 shows an alternative adhesive pattern, in which the central zone 34 is free of adhesive, and there are two relatively wide strips of adhesive 36 which stop short of the lateral edges of the article. In this case there is adhesive on the laterally inner part of each flap, but not on its laterally outer part. The drawings show, by way of example only, some measurements in millimetres for size of the adhesives stripes. The article itself has, in this case, an overall length of 227mm and overall width of 119mm.

The flaps of the article according to the present invention are so designed that, when the user has secured the article to the crotch region of a pair of panties, and has then pulled those panties on, the flaps automatically wrap themselves into the desired position overlying the exterior of the crotch region, without the user needing to take any additional action to achieve this. The reason why this is possible can be understood from a consideration of Figure 2, where additional lines 40 have been drawn in to indicate the edges of the crotch region of the panties. It is to be understood that the precise lines shown are not universally the same, and that there are, in particular, differences from one part of the world to another as regards the shape of panties which is normally preferred. However, the lines which are shown are fairly typical, and the principle which is being illustrated is of general applicability. It might, however, be desirable, in order to cater for panties with crotch regions differing greatly from what is shown, to make a corresponding difference to the shape of the sanitary napkin.

As can be seen in Figure 2, the lines 40 are each at least approximately in the form of a segment of an ellipse. As the user draws the panties with this sanitary napkin in it in an upward direction, the portions of the flaps 20a outwardly of the lines 40 are located against the wearer's thighs, and tend to fold downwards about the lines 40. Once these flap portions are oriented at 90° with respect to the general plane of the remainder of the article, they are unstable in their position and tend to continue their rotation, flipping under the crotch region of the panties. The panty fastening adhesive then causes the flaps to adhere to the underside of the crotch region. If the extent to which the flaps fold is not sufficient to produce this adhesion, or if the adhesion occurs to an insufficient extent, the user can effect it manually.

The presence of the undulations 22 in the flaps, and the consequent increase in the laterally outward peripheral length thereof, are critical in causing the above folding action to occur. It will be readily understood that although it is possible to fold a planar sheet about a rectilinear axis without puckering, the same is not possible if the axis about which folding is to take place is curved, unless the sheet is stretchable (which the material of the flaps in the article of the present invention is not). What makes the folding possible in the present case, without any puckering, is that the flaps are not planar. The non-planar nature of the flaps not only permits folding to take place about the lines 40, but also means that after the flaps have folded, and are lying against the underside of the crotch region of the panties, the increased length of the outer edge of the flaps enables those flaps to lie without substantial puckering against the underside of the crotch region.

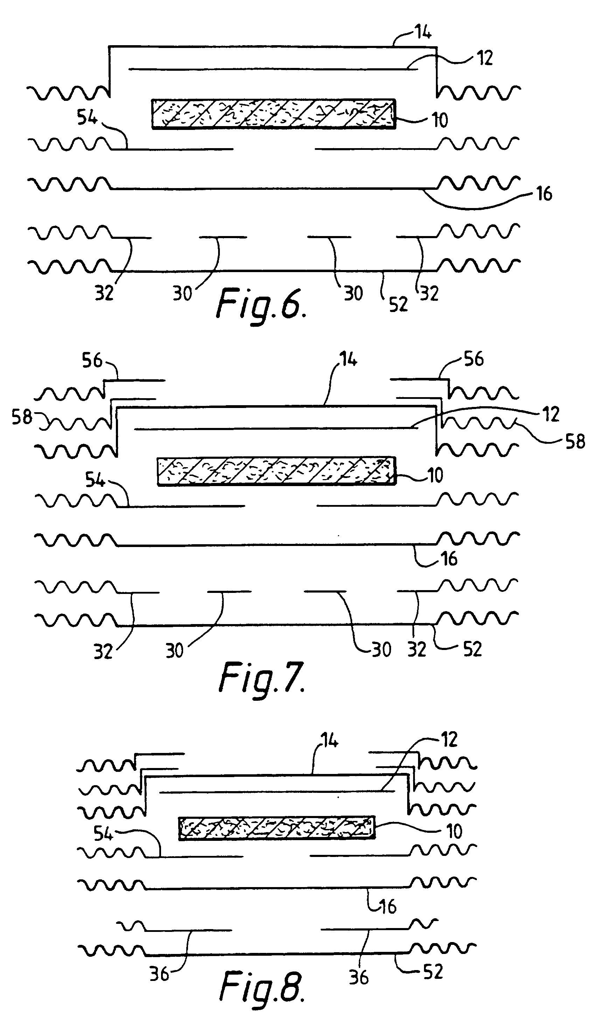

Figures 6 to 9 show a number of possible constructions for a sanitary napkin made according to the present invention. It is emphasised that the drawings are highly diagrammatic. Thus, for example, the waves which can be seen at the outer edge of some of the components, although representing the undulations 22, are shown for ease of illustration running at right angles to the actual orientation of the undulations 22. A number of the components in Figures 6 to 9 are common to what has been described above, and the same reference numerals are used. Additionally, however, each of Figures 6 to 9 uses the reference numeral 52 to denote the protective release layer, and the reference numeral 54 to denote the adhesive used to secure the topsheet and backsheet together.

The embodiment of Figure 7 also comprises two strips of soft non-woven material 56 which are secured by adhesive 58 to the surface of the topsheet 14, in the regions where the topsheet overlies the longitudinal edges of the core 10 and secondary topsheet 12. This is done in order to reduce the sharpness of this junction and increase the comfort to the wearer. The same is true in the case of the embodiment of Figure 8. The embodiments of Figures 6 and 7 use the adhesive pattern shown in Figure 4, whereas Figure 8 uses the adhesive pattern shown in Figure 5. The embodiment shown in Figure 9 is substantially the same as that shown in Figure 8, except that the topsheet 14 does not extend so far laterally outwards.

The sanitary napkin described above may be made of various materials, but, by way of example, the following Table sets out the construction of an embodiment of the present invention.

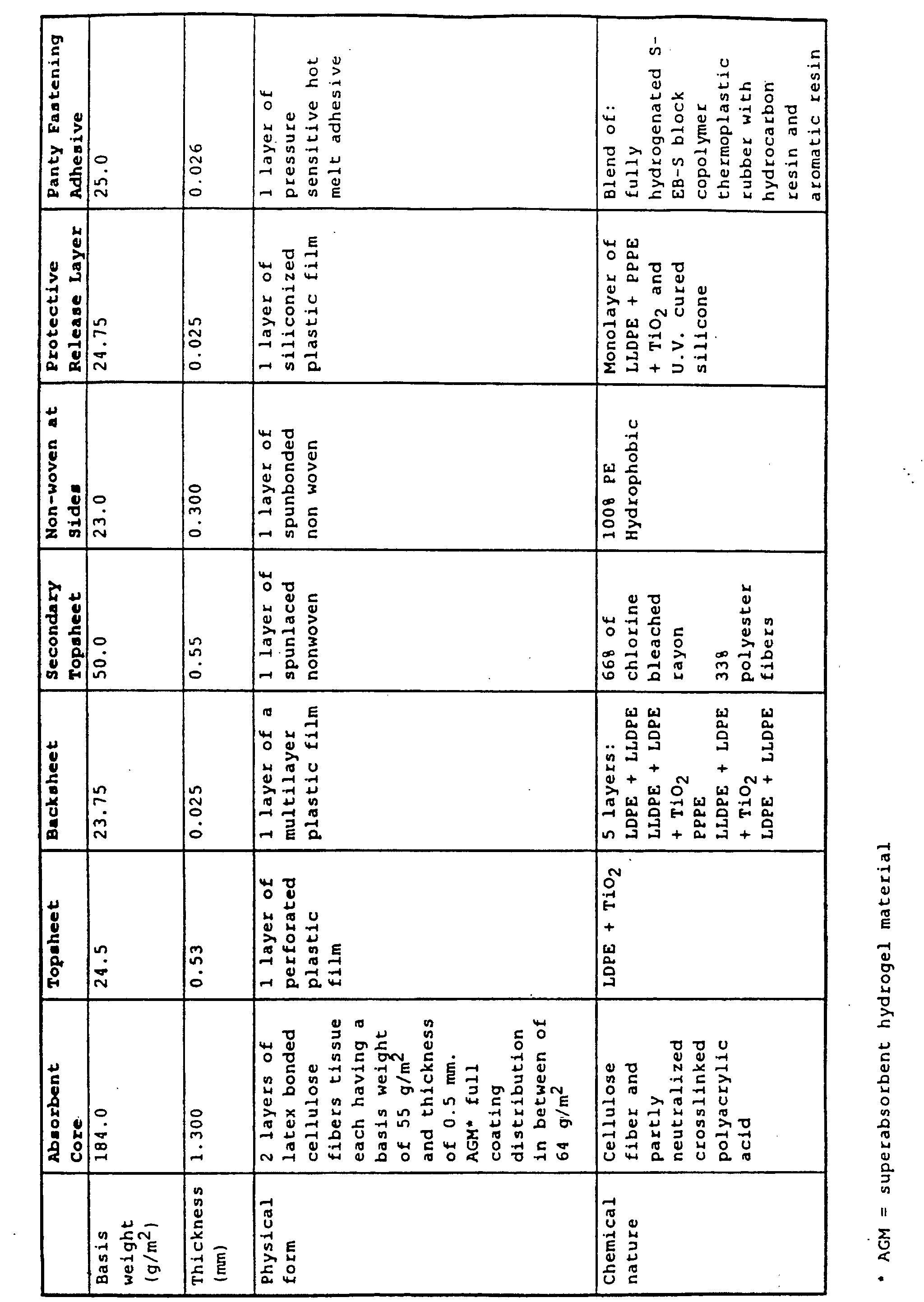

## Claims

1. A process for manufacturing absorbent sanitary article which comprises an absorbing region capable of absorbing body fluids and having a pair of side regions (20) which are to form side flaps (20a) located one on each of two opposite edges of the absorbing region, each flap (20a) being non-planar and having a first edge, nearer the absorbing region, which edge defines a non-rectilinear axis of rotation for the flap (20a) to pivot with respect to the absorbing region, and a second edge, remote from the said absorbing region, where the perimetral length is greater than for an equivalent planar flap, said process comprising the step of subjecting each of said regions (20) to a stretching force both in a longitudinal and in a transverse direction, in order to achieve the non-planar form of said flaps (20a).

2. A process according to claim 1, wherein the amount by which the perimetral length of each flap (20a) exceeds that of an equivalent planar flap is from 10-70%.

3. A process according to claim 2, wherein the said amount is from 15-50%.

4. A process according to claim 3, wherein the said amount is from 20-30%.

5. A process according to claim 4, wherein the said amount is about 25%.

6. A process according to any preceding claim, wherein the overall shape thereof is at least approximately that of an ellipse.

7. A process according to any preceding claim, wherein the absorbing region has side edges which are coincident with, or substantially parallel to, the said first edges of the flaps (20a).

8. A process according to claim 7, wherein the absorbing region has an hourglass shape.

9. A process according to any preceding claim, wherein each flap (20a) as seen in plan view, has its maximum width at a point intermediate its ends, and generally tapers from that point towards those ends.

10. A process according to any preceding claim, wherein the stretching force in a direction parallel to the width of the flaps (20a) results in an amount of stretch which increases, considered as a percentage of the unstretched width of the flaps (20a), from the region of maximum width towards the said ends.

11. A process according to claim 10, wherein the said amount of stretch parallel to the width of the flaps (20a) increases to a value of not more than 70% at the said ends.

12. A process according to claim 11, wherein the said amount of stretch parallel to the width of the flaps (20a) increases to a value of not more than 50% at the said ends.

13. A process according to claim 11 or 12, wherein the said amount of stretch parallel to the width of the flaps (20a) has a value of zero at the region of maximum width.

14. A process according to claim 11 or 12, wherein the said amount of stretch parallel to the width of the flaps (20a) has a value of at least 10% at the region of maximum width.

15. A process according to any preceding claims, wherein the stretching force in a direction parallel to the length of the flaps (20a) results in an amount of stretch which increases, considered as a percentage of the unstretched length of the flaps (20a), from the said first edge towards the said second edge.

16. A process according to claim 15, wherein the said amount of stretch parallel to the length of the flaps (20a) has a value of not more than 40% at the second edge.

17. A process according to claim 16, wherein the said amount of stretch parallel to the length of the flaps (20a) has a value of not more than 20% at the second edge.

18. A process according to claim 17, wherein the said amount of stretch parallel to the length of the flaps (20a) has a value of zero at the first edge.

19. A process according to any one of claims 15 to 18, wherein the average value of the said amount of stretch parallel to the length of the flaps (20a) is 10%.

20. An absorbent sanitary article manufactured according to the process of any of claims 1 to 19.

21. An article according to claim 20, wherein each flap (20a) has an undulating form and possesses a plurality of undulations (22) each of which has its crest running generally parallel to the width of the flap (20a).

22. An article according to claim 21, wherein the amplitude of each undulation (22) increases as considered in a direction from the said first edge towards the said second edge.

23. An article according to claim 21 or 22, wherein the undulations (22) are equidistant from one another.

24. An article according to any of claims 20 to 23, which comprises, considered from one face of the article to an opposite face, a liquid-impermeable backsheet (16), an absorbent core (10), and a liquid permeable topsheet (14).

25. An article according to claim 24, further comprising a liquid permeable secondary topsheet (12) located between the core (10) and the first mentioned topsheet (14).

26. An article according to claim 25, wherein at least the secondary topsheet (12) has an hourglass shape.

27. An article according to any one of claims 24 to 26, wherein a respective strip of soft material (56) is secured over the first mentioned topsheet (14) in each of the regions where the first mentioned topsheet (14) overlies a longitudinal edge of the core (10) and, if present, the secondary topsheet (12).

28. An article according to claim 27, wherein the said soft material (56) is a non-woven material.

29. An article according to any of claims 20 to 28 wherein a face thereof has adhesive (30, 32) thereon for securing the article to the clothing of a user.

30. An article according to claim 29, wherein the said adhesive is applied in a pattern which comprises two parallel stripes (30) spaced from a longitudinal centre line extending parallel to the length of the flaps (20a), and two laterally outward stripes (32), each spaced laterally outward from a respective one of the first mentioned stripes (30).

31. An article according to claim 30, wherein each of the laterally outward stripes (32) extends to a respective lateral edge of the article.

32. An article according to claim 29, wherein the said adhesive is applied in a pattern which comprises two parallel stripes (36) spaced from a longitudinal centre line extending parallel to the length of the flaps (20a), each stripe (36) covering at least part of a respective one of the flaps (20a).

33. An article according to claim 32, wherein neither stripe (36) extends to a respective lateral edge of the article.

## Patentansprüche

1. Verfahren zur Herstellung eines absorbierenden Hygieneartikels, welcher aufweist einen absorbierenden Bereich, der im Stande ist, Körperfluide zu absorbieren, und ein Paar Seitenbereiche (20), welche vorgesehen sind, um Seitenklappen (20a) zu bilden, wobei eine auf jedem der zwei gegenüberliegenden Ränder des absorbierenden Bereichs liegt, wobei jede Klappe (20a) nicht-planar ist und aufweist einen ersten Rand, der näher zu dem absorbierenden Bereich liegt, wobei der Rand eine nicht-geradlinige Schwenkachse für die Klappe (20a) bildet, um bezüglich des absorbierenden Bereichs zu schwenken, und einen zweiten Rand, der von dem absorbierenden Bereich entfernt ist, wo die Umfangslänge größer als für eine äquivalente planare Klappe ist, wobei das Verfahren den Schritt des Aussetzens von jedem der Bereiche (20) einer Streckkraft sowohl in einer longitudinalen als auch in einer transversalen Richtung aufweist, um die nicht-planare Form der Klappen (20a) zu erreichen.

2. Verfahren nach Anspruch 1, wobei der Betrag, um welchen die Umfangslänge jeder Klappe (20a) diejenige einer äquivalenten planaren Klappe überschreitet, von zehn bis 70% reicht.

3. Verfahren nach Anspruch 2, wobei der Betrag von 15 bis 50% reicht.

4. Verfahren nach Anspruch 3, wobei der Betrag von 20 bis 30% reicht.

5. Verfahren nach Anspruch 4, wobei der Betrag ungefähr 25% beträgt.

6. Verfahren nach einem der vorherigen Ansprüche, wobei dessen Gesamtform mindestens ungefähr der einer Ellipse ist.

7. Verfahren nach einem der vorherigen Ansprüche, wobei der absorbierende Bereich Seitenränder aufweist, welche mit den ersten Rändern der Klappen (20a) zusammenfallen oder im Wesentlichen parallel zu diesen sind.

8. Verfahren nach Anspruch 7, wobei der absorbierende Bereich eine Sanduhr-Form aufweist.

9. Verfahren nach einem der vorherigen Ansprüche, wobei jede Klappe (20a), betrachtet in der Draufsicht, ihre maximale Breite bei einem Punkt zwischen ihren Enden aufweist und sich im Allgemeinen von diesem Punkt in Richtung auf diese Enden verjüngt.

10. Verfahren nach einem der vorherigen Ansprüche, wobei die Streckkraft in einer zu der Breite der Klappen (20a) parallelen Richtung zu einem Streckbetrag führt, welcher, betrachtet als Prozentsatz der ungestreckten Breite der Klappen (20a), von dem Bereich maximaler Breite in Richtung auf die Enden ansteigt.

11. Verfahren nach Anspruch 10, wobei der Streckbetrag parallel zu der Breite der Klappen (20a) auf einen Wert von nicht mehr als 70% bei den Enden ansteigt.

12. Verfahren nach Anspruch 11, wobei der Streckbetrag parallel zu der Breite der Klappen (20a) auf einen Wert von nicht mehr als 50% bei den Enden ansteigt.

13. Verfahren nach Anspruch 11 oder 12, wobei der Streckbetrag parallel zu der Breite der Klappen (20a) einen Wert von 0 bei dem Bereich maximaler Breite aufweist.

14. Verfahren nach Anspruch 11 oder 12, wobei der Streckbetrag parallel zu der Breite der Klappen (20a) einen Wert von mindestens 10% bei dem Bereich maximaler Breite aufweist.

15. Verfahren nach einem der vorherigen Ansprüche, wobei die Streckkraft in einer zu der Länge der Klappen (20a) parallelen Richtung zu einem Streckbetrag führt, welcher, betrachtet als Prozentsatz der ungestreckten Länge der Klappen (20a), von dem ersten Rand in Richtung auf den zweiten Rand ansteigt.

16. Verfahren nach Anspruch 15, wobei der Streckbetrag parallel zu der Länge der Klappen (20a) einen Wert von nicht mehr als 40% bei dem zweiten Rand aufweist.

17. Verfahren nach Anspruch 16, wobei der Streckbetrag parallel zu der Länge der Klappen (20a) einen Wert von nicht mehr als 20% bei dem zweiten Rand aufweist.

18. Verfahren nach Anspruch 17, wobei der Streckbetrag parallel zu der Länge der Klappen (20a) einen Wert von 0 bei dem ersten Rand aufweist.

19. Verfahren nach einem der vorherigen Ansprüche 15 bis 18, wobei der durchschnittliche Wert des Streckbetrags parallel zu der Länge der Klappen (20a) 10% beträgt.

20. Absorbierender Hygieneartikel, der gemäß dem Verfahren von einem der Ansprüche 1 bis 19 hergestellt ist.

21. Artikel nach Anspruch 20, wobei jede Klappe (20a) eine gewellte Form aufweist und eine Vielzahl von Wellen (22) besitzt, wobei jede von diesen einen Kamm derart aufweist, dass er im Allgemeinen parallel zu der Breite der Klappe (20a) verläuft.

22. Artikel nach Anspruch 21, wobei die Amplitude von jeder Welle (22) betrachtet in einer Richtung von dem ersten Rand in Richtung auf den zweiten Rand ansteigt.

23. Artikel nach Anspruch 21 oder 22, wobei die Wellen (22) äquidistant zueinander sind.

24. Artikel nach Ansprüchen 20 bis 23, welcher umfasst, betrachtet von einer Seite des Artikels zu einer gegenüberliegenden Seite, eine flüssigkeitsundurchlässige Außenlage (16), einen absorbierenden Kern (10) und eine flüssigkeitsdurchlässige Decklage (14).

25. Artikel nach Anspruch 24, ferner umfassend eine flüssigkeitsdurchlässige sekundäre Decklage (12), die sich zwischen dem Kern (10) und der erst-erwähnten Decklage (14) befindet.

26. Artikel nach Anspruch 25, wobei mindestens die sekundäre Decklage (12) eine Sanduhr-Form aufweist.

27. Artikel nach einem der Ansprüche 24 bis 26, wobei ein entsprechender Streifen aus weichem Material (56) über der erst-erwähnten Decklage (14) in jedem der Bereiche befestigt ist, wo die erst-erwähnte Decklage (14) über einem longitudinalen Rand des Kerns (10) und, falls vorhanden, der sekundären Decklage (12) liegt.

28. Artikel nach Anspruch 27, wobei das weiche Material (56) ein Nonwoven-Material ist.

29. Artikel nach einem der Ansprüche 20 bis 28, wobei eine Seite von diesem einen Klebstoff (30, 32) darauf zum Befestigen des Artikels an der Kleidung eines Benutzers aufweist.

30. Artikel nach Anspruch 29, wobei der Klebstoff in einem Muster aufgebracht ist, welches umfasst zwei parallele Streifen (30), die von einer zu der Länge der Klappen (20a) parallel verlaufenden, longitudinalen Mittellinie beabstandet sind, und zwei seitlich äußere Streifen (32), von denen seitlich nach außen von einem entsprechenden der erst-erwähnten Streifen (30) beabstandet ist.

31. Artikel nach Anspruch 30, wobei jeder der seitlich äußeren Streifen (32) zu einem entsprechenden lateralen Rand des Artikels läuft.

32. Artikel nach Anspruch 29, wobei der Klebstoff in einem Muster aufgebracht ist, welches umfasst zwei parallele Streifen (36), die von einer zu der Länge der Klappen (20a) parallel verlaufenden longitudinalen Mittellinie beabstandet sind, wobei jeder Streifen (36) mindestens einen Abschnitt einer entsprechenden der Klappen (20a) bedeckt.

33. Artikel nach Anspruch 32, wobei kein Streifen (36) zu einem entsprechenden lateralen Rand des Artikels verläuft.

## Revendications

1. Procédé de fabrication d'un article hygiénique absorbant qui comprend une région absorbante capable d'absorber les fluides corporels et ayant une paire de régions latérales (20) qui sont destinées à former des rabats latéraux (20a) situés sur chacun des deux bords opposés de la région absorbante, chaque rabat (20a) étant non plat et ayant un premier bord, plus proche de la région absorbante, lequel bord définit un axe de rotation non rectiligne pour que le rabat (20a) pivote par rapport à la région absorbante, et un second bord, éloigné de ladite région absorbante, dont la longueur du périmètre est supérieure à celle d'un rabat plat équivalent, ledit procédé comprenant l'étape consistant à soumettre chacune desdites régions (20) à une force d'étirement à la fois dans une direction longitudinale et dans une direction transversale, afin d'obtenir une forme non plat desdits rabats (20a).

2. Procédé selon la revendication 1, dans lequel la proportion dans laquelle la longueur du périmètre de chaque rabat (20a) dépasse celle d'un rabat plat équivalent est de 10-70 %.

3. Procédé selon la revendication 2, dans lequel ladite proportion est de 15-50 %.

4. Procédé selon la revendication 3, dans lequel ladite proportion est de 20-30 %.

5. Procédé selon la revendication 4, dans lequel ladite proportion est d'environ 25 %.

6. Procédé selon l'une quelconque des revendications précédentes, dans lequel la forme globale de l'article est au moins approximativement celle d'une ellipse.

7. Procédé selon l'une quelconque des revendications précédentes, dans lequel la région absorbante a des bords latéraux qui coïncident avec, ou sont essentiellement parallèles auxdits premiers bords des rabats (20a).

8. Procédé selon la revendication 7, dans lequel la région absorbante a la forme d'un verre de montre.

9. Procédé selon l'une quelconque des revendications précédentes, dans lequel chaque rabat (20a), comme on le voit sur la vue en plan, atteint sa largeur maximale au milieu de ses extrémités, laquelle diminue généralement de ce point vers ces extrémités.

10. Procédé selon l'une quelconque des revendications précédentes, dans lequel la force d'étirement dans une direction parallèle à la largeur des rabats (20a) a pour résultat une valeur d'étirement qui augmente, exprimée en pourcentage de la largeur non étirée des rabats (20a), à partir de la région de largeur maximale vers lesdites extrémités.

11. Procédé selon la revendication 10, dans lequel ladite valeur d'étirement parallèle à la largeur des rabats (20a) augmente jusqu'à une valeur non supérieure à 70 % auxdites extrémités.

12. Procédé selon la revendication 11, dans lequel ladite valeur d'étirement parallèle à la largeur des rabats (20a) augmente jusqu'à une valeur non supérieure à 50 % auxdites extrémités.

13. Procédé selon la revendication 11 ou 12, dans lequel ladite valeur d'étirement parallèle à la largeur des rabats (20a) a une valeur de zéro dans la région de largeur maximale.

14. Procédé selon la revendication 11 ou 12, dans lequel ladite valeur d'étirement parallèle à la largeur des rabats (20a) a une valeur d'au moins 10 % dans la région de largeur maximale.

15. Procédé selon l'une quelconque des revendications précédentes, dans lequel la force d'étirement dans une direction parallèle à la longueur des rabats (20a) a pour résultat une valeur d'étirement qui augmente, exprimée en pourcentage de la longueur non étirée des rabats (20a), à partir dudit premier bord vers ledit second bord.

16. Procédé selon la revendication 15, dans lequel ladite valeur d'étirement parallèle à la longueur des rabats (20a) a une valeur non supérieure à 40 %, au second bord.

17. Procédé selon la revendication 16, dans lequel ladite valeur d'étirement parallèle à la longueur des rabats (20a) a une valeur non supérieure à 20 %, au second bord.

18. Procédé selon la revendication 17, dans lequel ladite valeur d'étirement parallèle à la longueur des rabats (20a) a une valeur de zéro, au premier bord.

19. Procédé selon l'une quelconque des revendications 15 à 18, dans lequel la moyenne de ladite valeur d'étirement parallèle à la longueur des rabats (20a) est de 10 %.

20. Article hygiénique absorbant fabriqué conformément au procédé de l'une quelconque des revendications 1 à 19.

21. Article selon la revendication 20, dans lequel chaque rabat (20a) a une forme ondulée et possède une pluralité d'ondulations (22), dont chacune présente une crête s'étendant généralement parallèlement à la largeur du rabat (20a).

22. Article selon la revendication 21, dans lequel l'amplitude de chaque ondulation (22) augmente, telle que considérée dans une direction partant dudit premier bord vers ledit second bord.

23. Article selon la revendication 21 ou 22, dans lequel les ondulations (22) sont équidistantes l'une de l'autre.

24. Article selon l'une quelconque des revendications 20 à 23, qui comprend, si l'on considère une face avant de l'article par rapport à une face opposée, une feuille de fond imperméable aux liquides (16), un coeur absorbant (10), et une feuille supérieure perméable aux liquides (14).

25. Article selon la revendication 24, comprenant en outre une feuille supérieure secondaire perméable aux liquides (12) située entre le coeur (10) et la première feuille supérieure mentionnée (14).

26. Article selon la revendication 25, dans lequel au moins la feuille supérieure secondaire (12) a la forme d'un verre de montre.

27. Article selon l'une quelconque des revendications 24 à 26, dans lequel une bande respective de matière molle (56) est fixée sur la première feuille supérieure mentionnée (14) dans chacune des régions où la première feuille supérieure mentionnée (14) recouvre un bord longitudinal du coeur (10) et, si elle est présente, la feuille supérieure secondaire (12).

28. Article selon la revendication 27, dans lequel ladite matière molle (56) est une matière non tissée.

29. Article selon l'une quelconque des revendications 20 à 28, dans lequel une face de celui-ci est recouverte d'un adhésif (30, 32) pour fixer l'article au vêtement d'une utilisatrice.

30. Article selon la revendication 29, dans lequel ledit adhésif est appliqué suivant un motif qui comprend deux bandes parallèles (30) espacées de la ligne centrale longitudinale s'étendant de manière parallèle à la longueur des rabats (20a), et deux bandes s'étendant latéralement vers l'extérieur (32), espacées chacune latéralement vers l'extérieur par rapport à l'une des premières bandes mentionnées (30), respectivement.

31. Article selon la revendication 30, dans lequel chaque bande s'étendant latéralement vers l'extérieur (32) s'étend vers un bord latéral respectif de l'article.

32. Article selon la revendication 29, dans lequel ledit adhésif est appliqué suivant un motif qui comprend deux bandes parallèles (36) espacées d'une ligne centrale longitudinale s'étendant de manière parallèle à la longueur des rabats (20a), chaque bande (36) recouvrant au moins une partie de l'un des rabats (20a), respectivement.

33. Article selon la revendication 32, dans lequel aucune bande (36) ne s'étend vers un bord latéral respectif de l'article.
